# EUROPÄISCHE PATENTSCHRIFT

(11) **EP 1 276 416 B1**
(45) Veröffentlichungstag und Bekanntmachung des Hinweises auf die Patenterteilung: **25.05.2005**
(21) Anmeldenummer: 01927916.5
(22) Anmeldetag: 19.04.2001
(51) Int. Cl.: A61B 5/11, A61B 5/113

(54) **VORRICHTUNG ZUR ERZEUGUNG VON MESSDATEN BETREFFEND BEWEGUNGEN DER BAUCHDECKE**
DEVICE FOR PRODUCING MEASURING DATA RELATING TO THE MOVEMENTS OF THE ABDOMINAL WALL
DISPOSITIF POUR CREER DES DONNEES DE MESURES CONCERNANT LES MOUVEMENTS DE LA PAROI ABDOMINALE

(30) Priorität: 19.04.2000 DE 10019634
(43) Veröffentlichungstag der Anmeldung: 22.01.2003
(73) Patentinhaber: Friendly Sensors AG, 07743 Jena (DE)
(72) Erfinder: FRIEDRICHS, Arnd, 07743 Jena (DE)
(74) Vertreter: Behrmann, Niels, Dipl.-Ing.
(86) Internationale Anmeldenummer: PCT/EP2001/004441
(87) Internationale Veröffentlichungsnummer: WO 2001/080737

(56) Entgegenhaltungen:
- EP-A- 0 752 233
- WO-A-98/29035
- US-A- 3 606 879
- US-A- 5 588 439
- US-A- 5 829 438
- LIN W C ET AL: "A microprocessor-based data acquisition and processing system for studying the kinematics of labor" PROCEEDINGS OF THE IEEE, MAY 1977, USA, Bd. 65, Nr. 5, Seiten 722-729, XP002177129 ISSN: 0018-9219

## Beschreibung

Die vorliegende Erfindung betrifft eine Vorrichtung zur Erzeugung von Messdaten betreffend Bewegungen der Bauchdecke einer Person. Eine derartige Vorrichtung als allgemeines Verfahren zur Erfassung von Körperbewegungen ist aus der DE 42 14 523 A1 bekannt, wobei diese bekannte Vorrichtung primär dazu dient, Zustände und Veränderungen des Haltungs- und Bewegungsapparates des Menschen zu überwachen.

Eine sehr spezielle Form von Körperbewegungen sind die durch Wehen hervorgerufenen Kontraktionen und Dilatationen der Bauchdecke einer Schwangeren. Bekannte Verfahren zur Messung von Wehen basieren auf der Messung des Bauchumfanges: Zu diesem Zweck wird ein Band um den Bauch gelegt, dessen Länge bzw. Spannungszustand kontinuierlich erfasst wird und etwa durch sog. Wehenschreiber schriftlich auf Endlospapier eingetragen wird.

Derartige bekannte Verfahren weisen allerdings das Problem auf, dass außer eher summarischer Erfassung des Bauchumfanges keine Rückschlüsse auf einen lokalen Verlauf einer Wehe möglich sind: Zwar wird der Normalfall einer Wehe, d. h. die Veränderung der Bauchdecke durch repetitive Muskelverkürzung der Uterus-Gesamtmasse, hinreichend genau erfasst, insbesondere jedoch pathologische Wehenverläufe bzw. das Erkennen sog. physiologischer Übergangsformen ist mit bekannten, auf Dehnung eines Bandes oder Bauchwand-Spannungsänderung, erfaßt mittels Druckwandlerbasierenden Verfahren ("Tokographie") schwierig.

Während nämlich eine normale Uteruskontraktion im allgemeinen im Bereich der rechten oder linken Tubenecke beginnt und sich von dort koordiniert innerhalb einer Zeit von etwa 15 Sekunden über den ganzen Uterus ausbreitet, können gerade bei abweichenden Erregungsabläufen (etwa drohende Frühgeburt) andere Wehenformen auftreten, charakterisiert etwa durch auf einzelne Uterusbereiche begrenzte Wehen, oder durch unkoordinierte Teilkontraktionen.

Aus der WO 98/29035 ist eine Vorrichtung zur Erzeugung von Messdaten betreffend Bewegungen der Bauchdecke einer Person bekannt. Diese Vorrichtung weist eine oder mehrere Sensoreinheiten in Form sog. maternal probes auf, die zum lösbaren Befestigen auf der Haut an einem Bauchbereich einer Person ausgebildet sind. Zwischen diesen maternal probes und sog. Referenzelementen, welche in der Nähe des Körpers der Person, jedoch außerhalb des Bauchbereiches und isoliert von Bewegungen der Person befestigt sind, erfolgt dann eine Erfassung elektrisch auswertbarer Signale zum Erzeugen eines den jeweiligen Abstand sowie Änderungen desselben entsprechenden Abstandssignals.

Der so gebildeten Messeinrichtung ist gemäß der Lehre der WO 98/29035 eine Auswerteeinheit nachgeschaltet, die zum Auswerten impuls- und/oder wellenförmiger Änderungen des Abstandssignals dergestalt ausgebildet ist, dass nicht-periodische oder periodische Signaländerungen einer Dauer von mindestens zehn Sekunden erfasst, von periodischen, durch eine menschliche Atmung bestimmten Änderungen unterschieden und als elektronisch weiter auswertbares Anzeigesignal ausgegeben werden können

Aufgabe ist es daher, eine verbesserte Vorrichtung zur Erzeugung von Messdaten betreffend Bauchdeckenbewegungen, insbesondere auch zur Wehenerkennung, zu schaffen, welche die Nachteile bekannter Vorrichtungen zur Wehenmessung überwindet und insbesondere die Möglichkeit bietet, aus Bauchdeckenbewegungen einer Schwangeren differenzierte Messwerte über Art und Charakter der Wehentätigkeit zu erhalten, auch mit dem Zweck, atypische Wehentätigkeit zu detektieren, damit einer möglichen Frühgeburt rechtzeitig (etwa medikamentös) entgegenwirken zu können und so die medizinische Betreuung von Risikoschwangerschaften beträchtlich zu erleichtern.

Diese Aufgabe stellt sich vor dem Hintergrund, dass bei etwa 800 000 Geburten pro Jahr in Deutschland ca. 6 bis 7 % als sog. Frühgeburten (also vor dem medizinisch als normal eingestuften Toleranzbereich um den vorbestimmten Geburtstermin herum) auftreten und üblicherweise davon ausgegangen wird, dass bei ca. 15 % aller Schwangerschaften eine wehenhemmende medikamentöse Therapie angezeigt ist.

Die Erfindung ist in den Ansprüchen definiert.

So basiert das erfindungsgemäße Prinzip zur Messdatenerfassung der Bauchdecke auf einer Entfernungsmessung zwischen den beiden Sensoreinheiten des auf der Haut in dem ersten (bzw. zweiten) Abstand aufgebrachten Paares von Sensoreinheiten, wobei bevorzugt diese Entfernungsbestimmung elektronisch durch ein Ultraschall- bzw. elektromagnetisches Sender-Empfänger-System realisiert ist, wie es in der gattungsbildenden DE 42 14 523 beschrieben ist. Insbesondere hat sich für die Realisierung der vorliegenden Erfindung das Prinzip der Ultraschall-Abstandsmessung bewährt, bei welchem die durch Bewegungen der Bauchdecke bewirkten Laufzeitunterschiede im Ultraschallsignal zwischen den Sensoreinheiten gemessen und ausgewertet werden (alternativ ist es bevorzugt, Phasendifferenzen zwischen gesendetem und empfangenem Signal zu verwenden).

Gemäß der vorliegenden Erfindung findet nunmehr diese bekannte Technologie Anwendung auf die besonderen Anforderungen der Wehenmessung, wobei, zusammen mit der erfindungsgemäß vorgesehenen Auswerteeinheit, gerade die abstandsabhängige Messdatenerfassung von Bewegungen der Bauchdecke sich als besonderes zuverlässig und präzise herausgestellt hat, um nicht nur generell auf das Vorliegen einer Wehe zu schließen (welches erfindungsgemäß durch die mit der Auswerteeinheit realisierte signalbezogene bzw. zeitbezogene Diskriminierung erfolgt), auch bietet insbesondere das weiterbildungsgemäße Vorsehen einer Mehrzahl von Sensorpaaren die Möglichkeit, die zeitliche und/oder örtlich/räumliche Verteilung von durch Wehen induzierten Bauchdeckenbewegungen präzise zu erfassen und so einer Diagnose daraufhin zuzuführen, dass es sich um die reguläre, die eine therapeutischen Eingriff erfordert (vorzeitiger Eintritt einer koordinierten Wehentätigkeit), oder aber um unkoordinierte Kontraktionen handelt.

Zudem hat es sich im Rahmen der Erfindung herausgestellt, dass die eigentliche Signalform regulärer Wehen eine symmetrische im Hinblick auf das Signal-Zeitdiagramm ist, also ansteigende und abfallende Flanke eines durch Abstandsänderung gemessenen Wehensignals bezogen auf einen Mittelwert symmetrisch liegen. Weiterbildungsgemäß sind daher die Signalformerfassungsmittel vorgesehen, die, zur Abgrenzung von anderen Signalen (etwa durch Sensorbewegung erzeugte Artefakte, Kindsbewegungen oder Atembewegungen) mit hoher Genauigkeit und Störunempfindlichkeit das Vorliegen von Wehentätigkeit erkennen.

Gemäß einer bevorzugten Weiterbildung (best mode) sind mindestens zwei Paare von Sensoreinheiten vorgesehen, die jeweils eine sich kreuzende Strecke aufspannen (d. h. einen in etwa rechtem Winkel ausbilden). Besonders bevorzugt lassen sich so die unterschiedlichen Richtungen der Bauchdeckenbewegung berücksichtigen und etwa durch Summen- bzw. Differenzbildung der aus beiden Sensorpaaren erhaltenen Signale für eine noch genauere Messung auswerten.

Weitergebildet werden kann diese Ausführungsform dadurch, dass noch weitere Sensorpaare kreuzend auf der Bauchdecke angeordnet sind, so dass die Bauchdecke im Hinblick auf konkrete Orte einer Bewegung (und damit Kontraktion des unterliegenden Uterus) gezielt beobachtet und ausgewertet werden kann.

Besonders bevorzugt ist es zudem, neben dem erfindungsgemäß erfassten und ausgegebenen Signal zusätzlich ein Atmungssignal der überwachten Person zu erfassen, wobei dies im Rahmen der Erfindung ebenfalls durch Auswertung des durch die erfindungsgemäße längenabhängige Abstandsmessung gewonnenen Signal geschieht: Auch Atmen führt zu (mit der Atmungsfrequenz von ca. 0,2 Hz) periodischen Bewegungen der Bauchdecke, wobei jedoch diese Signale im Rahmen der Erfindung zuverlässig von den für Wehen charakteristischen Abstandssignalen diskriminiert werden. Die zusätzlich aktive Überwachung des Atemsignals ermöglicht es daher nicht nur, die Qualität des eigentlichen Wehensignals zu verbessern, sondern zusätzlich auch kritische Zustände, etwa das Ausbleiben von Atemsignalen (Schlafapnoezustände, Hyperventilatonszustände) zuverlässig zu erfassen und entsprechende Gegenmaßnahmen zu ergreifen.

Besonders geeignet ist die vorliegende Erfindung zur Verwendung mit einer portablen, und weiter bevorzugt drahtlos datenmäßig mit einer Basisstation zusammenarbeitenden Einheit: Die vorliegende Erfindung ermöglicht es nämlich, die erfindungsgemäß zur Messdatenerfassung und -auswertung vorgesehenen, üblicherweise mittels geeignet programmierten Controllern realisierten Einheiten in einem tragbaren, batteriebetriebenen Gehäuse vorzusehen, welches von der Schwangeren kontinuierlich mitgeführt wird, und welches das permanente Überwachen auf Wehentätigkeit rund um die Uhr, eingeschlossen das Ermitteln eines Wehenprofils, das Beobachten der Abstände zwischen den Wehen usw., ermöglicht.

Insbesondere im Hinblick auf die für eine Schwangere teilweise sehr belastende Zeit vor der Geburt gestattet es damit die vorliegende Erfindung, nicht nur ohne stationäre Anbindung an einen bekannten Wehenschreiber eine zuverlässige Überwachung der Wehentätigkeit zu erhalten, zusätzlich wird neben einem beträchtlichen Komfortgewinn das Sicherheitsgefühl der Schwangeren erhöht.

Im Ergebnis wird mit der vorliegenden Erfindung ein Instrument bzw. ein Verfahren geschaffen, welches nicht nur geeignet ist, kritische von normalen Wehenzuständen schnell und zuverlässiger zu unterscheiden (und damit sowohl schnelle Hilfe leisten zu können, als auch überflüssige Medikamentendarreichung zu vermeiden), durch die portable Realisierung der Erfindung ist zudem in der Überwachung der Schangerschaft ein überaus vorteilhaftes Instrument zur Flexiblisierung und zur Komforterhöhung geschaffen.

Weitere Vorteile, Merkmale und Einzelheiten der Erfindung ergeben sich aus der nachfolgenden Beschreibung bevorzugter Ausführungsbeispiele sowie anhand der Zeichnungen; diese zeigen in:
- Fig. 1:: eine schematische Ansicht der erfindungsgemäßen Vorrichtung zur Erzeugung von Messdaten an einer Position der Bauchdecke mit einem einzelnen Paar von Sensoreinheiten sowie nachgeschalteten Funktionseinheiten;
- Fig. 2:: eine Prinzipansicht als Draufsicht auf die Bauchdecke mit der Position der Sensoreinheiten;
- Fig. 3:: eine alternative Ausführungsform mit zwei Paaren von Sensoreinheiten sowie einer nach geschalteten portablen Mess- und Übertragungsvorrichtung;
- Fig. 4:: ein Signal-Zeitdiagramm von den zwei in Fig. 3 gezeigten Sensorpaaren als Einzelsignale;
- Fig. 5:: ein Differenzsignal der Einzelsignale aus Fig. 4;
- Fig. 6,7:: eine Verdeutlichung der Verschiebung der Positionen und Abstände der jeweiligen · Sensorpaare als Reaktion auf eine Wehe und
- Fig. 8,9:: beispielhafte Konfigurationen bei der Anordnung einer Mehrzahl von Sensorpaaren zur Erfassung einer örtlichen Verteilung von Bewegungs- bzw. Wehenaktivität auf der Bauchdecke.

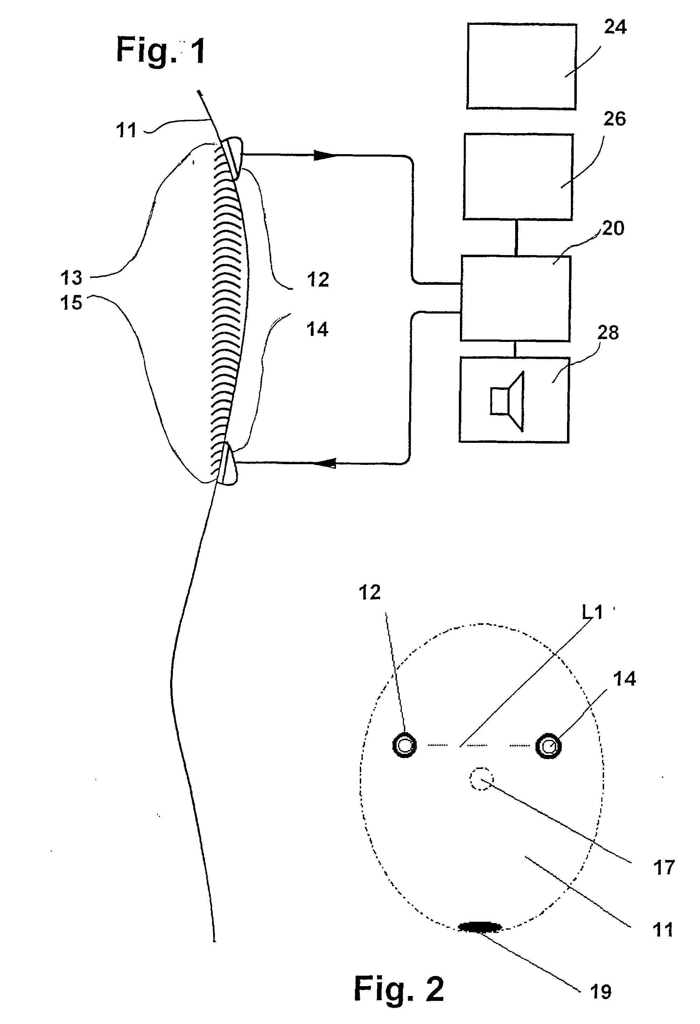

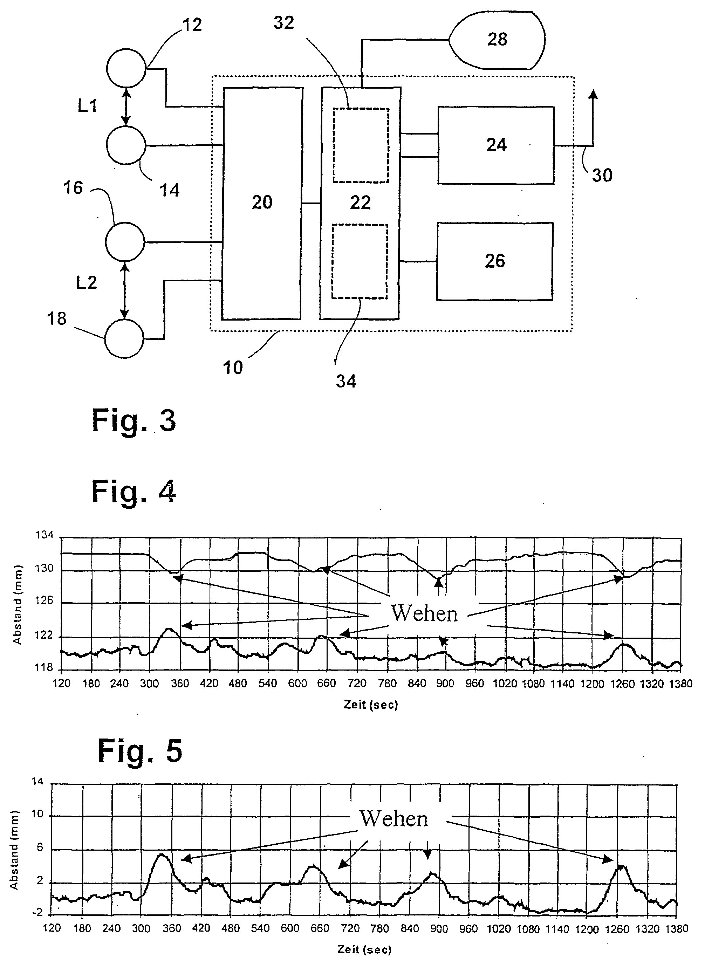

Fig. 1 zeigt schematisch, wie auf einer Bauchdecke 11 einer Schwangeren eine erste Sensoreinheit 12 und eine zweite Sensoreinheit 14 in einem vorbestimmten Abstand L1 (Fig. 3) voneinander beabstandet aufgesetzt sind; an jeweiligen Sensorpositionen 13 bzw. 15.

Die Sensoreinheiten 12, 14 sind Sender und Empfänger einer Ultraschall-Entfernungsmesseinheit 20 zugeordnet, die in ansonsten bekannter Weise auf der Basis von Laufzeitunterschieden bei Abstandsänderung des Abstandes L1 zwischen den Sensoreinheiten 12, 14 (bzw. die Positionen 13, 15) ein entsprechendes, zur weiteren Auswertung geeignetes Signal ausgibt.

Konkret ist, wie in der Fig. 1 gezeigt, der Entfernungsmesseinheit 20 in schematischer Weise eine Auswerte- und Ausgabeeinheit 28 nachgeschaltet, die als Reaktion auf das Entfernungsmesssignal der Einheit 20 auf das charakteristische Vorliegen einer Wehe schließt, insbesondere nämlich dadurch, dass eine Signaländerung des Entfernungssignals über einen Zeitraum typischerweise größer 30 Sekunden, üblicherweise im Bereich zwischen 1 und 1,5 Minuten, einer vorbestimmten Mindestamplitude vorliegt und entsprechend ein Wehensignal ausgibt. Parallel wird ein Wehensignal in einer Auswert- und Speichereinheit 26 gespeichert, insbesondere auch mit dem Zweck, Abstände zwischen aufeinanderfolgenden Wehen zu überwachen, und über eine lediglich schematisch gezeigte Kommunikationseinheit 24 findet eine Anbindung des wie in Fig. 1 gezeigten, auf einfachstem Weg realisierten Wehenmonitors an eine drahtlos angebundene Basisstation zur weiteren Überwachung und Auswertung statt.

Die Fig. 2 verdeutlicht in diesem -- einfachsten -- Anwendungsfall (d. h. bei lediglich einem Paar von Sensoreinheiten), wie zwischen den einzelnen Sensoren 12, 14 eine Strecke L1 gebildet ist, deren Länge sich in Abhängigkeit von der wehenabhängigen Bauchdeckenbewegung der Schwangeren ändert und im Rahmen der vorliegenden Erfindung ausgewertet werden kann. In der Fig. 2 zeigt das Bezugszeichen 17 symbolisch den Bauchnabel, das Bezugszeichen 19 die Symphyse.

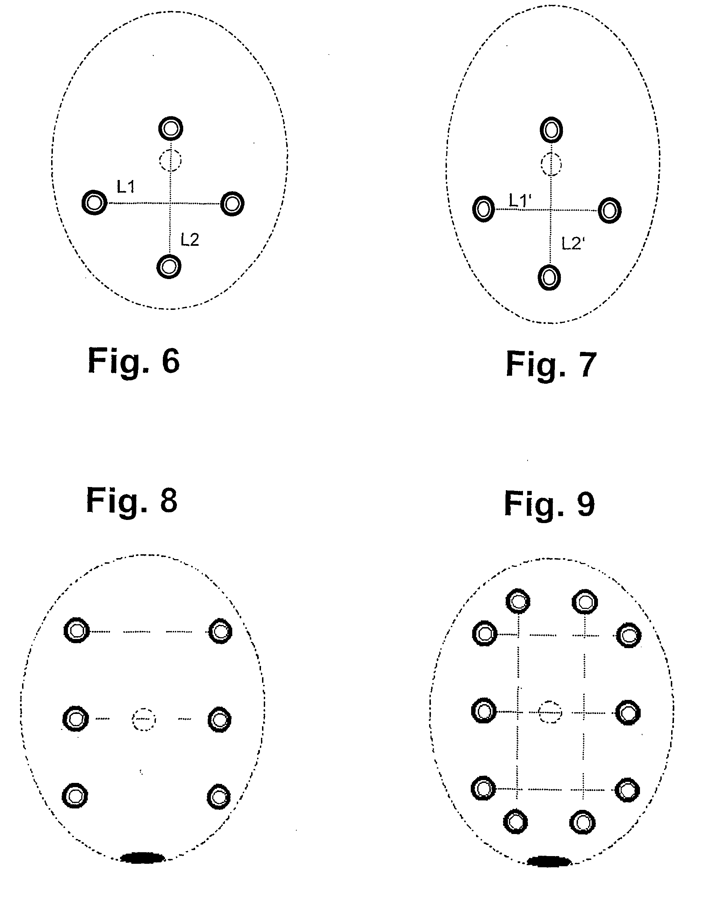

Unter Bezug auf die Fig. 3 wird eine weitere Ausführungsform der vorliegenden Erfindung beschrieben; in diesem Ausführungsbeispiel wird das erste Paar von Sensoreinheiten 12, 14 ergänzt um ein zweites Paar von Sensoreinheiten 16, 18, die einen zweiten Abstand L2 voneinander beabstandet sind, und wobei die Einheiten beispielsweise in der in Fig. 6 gezeigten Weise so auf der Bauchdecke 11 angeordnet sind, dass sich die gedachten Strecken L1, L2 zwischen den jeweiligen Sensoreinheitenpaaren kreuzen.

Wie in Fig. 3 gezeigt, empfängt die Ultraschall-Entfernungsmesseinheit 20 die Abstandssignale beider Sensoreinheitenpaare, mithin also zwei Einzelsignale, die exemplarisch in Fig. 4 als Signal-Zeitdiagramm gezeigt sind: Der obere, mit dünner Linie gezeigte Kurvenverlauf repräsentiert den Signalverlauf zwischen dem zweiten Sensorpaar 16, 18, also die Veränderung des Abstande L2, während die untere, mit dicker Linie gezeichnete Signalkurve dem Abstand L1 zwischen dem ersten Sensorpaar 12, 14 entspricht. Charakteristische Wehentätigkeit ist durch die entsprechenden Pfeile eingezeichnet, wobei sich zeigt, dass ein typisches Wehensignal auf beiden Kanälen (12/14, 16/18) mindestens 30, typischerweise 60 bis 90 Sekunden lang ist, eine im Zeitdiagramm i. w. symmetrische Kurvenform aufweist und in Abständen auftritt, die deutlich länger als das eigentliche Wehensignal sind.

Eine in Fig. 3 der Messeinheit 20 nachgeschaltete Auswerteeinheit 22 ist zur Aufbereitung und Analyse des (gegenüber Fig. 1 komplexeren) Signalmusters vorgesehen und gibt in der vorbeschriebenen Weise als Reaktion auf eine erfasste Wehentätigkeit ein Signal an die Ausgabeeinheit 28 aus. Zusätzlich wird die Kommunikationseinheit 24 (mit symbolischer Antenne 30 als Funk- bzw. geeignet konfiguriertem Mobiltelefoneinheit realisiert) angesteuert, ebenso wie die Speichereinheit 26 zum Abspeichern des erfassten Signalmusters sowie zum Beobachten und Analysieren aufeinanderfolgender Wehensignale.

Wie in der Fig. 5 gezeigt, ist die Auswerteeinheit 22 gemäß Fig. 3 in der Lage, nicht nur die beiden Messkanäle 12/14 bzw. 16/18 diskret auf Wehentätigkeit zu beobachten und auszuwerten, auch wird ein Differenzsignal dieser beiden Signale gebildet, welches dann gemäß Fig. 5 in weitaus deutlicherer Weise noch charakteristische Wehen zeigt; durch diese Maßnahme der Signalweiterverarbeitung lässt sich daher die Auflösung und Erkennungsgenauigkeit von Wehen bzw. von wehencharakteristischen Bauchdeckenbewegungen weiter erhöhen.

Die Fig. 3 verdeutlicht zusätzlich mit Funktionseinheiten 32, 34, dass eine Detailanalyse der empfangenen Signalverläufe im Hinblick auf wehenspezifische Signalmuster erfolgen kann (Einheit 32), sowie zusätzlich eine Überwachung der gemessenen Entfernungs- bzw. Abstandssignale auf Atmungssignale (Einheit 34) möglich ist: Diese treten typischerweise in einer Frequenz von etwa 0,2 Hz auf und sind ebenfalls als charakteristische (und natürlich gegenüber Wehen kurze) Bewegungen der Bauchdecke in der Signalform gemäß Fig. 4, Fig. 5 zu erkennen.

Die in Fig. 3 gezeigte Ausführungsform ermöglicht es daher in besonders günstiger Weise, zusätzlich zu der Wehenüberwachung auch noch eine Überwachung auf Atmungstätigkeit der Schwangeren vorzunehmen, wodurch nicht nur die eigentliche Qualität der Wehenerkennung erhöht, sondern zusätzlich die Atmung auf kritische Zustände überwacht werden kann; mithin also der erreichbare Sicherungsgrad der erfindungsgemäßen Vorrichtung beträchtlich erhöht wird.

Weiterbildungsgemäß ist die vorliegende Erfindung geeignet, Abstands- bzw. Bewegungssignale einer Vielzahl von Sensorpaaren zu erfassen. So stellt zwar die in Fig. 6, Fig. 7 gezeigte Konfiguration eine besonders geeignete, zweikanalige Auswertung (etwa gemäß Fig. 3 bis 5) dar, wobei die Fig. 7 typische Längenänderung L1', L2' der überwachten Abstände L1, L2 im Falle von Wehentätigkeit zeigt (symbolisch ist auch die Bauchdecke etwas gestreckt dargestellt), prinzipiell sind jedoch beliebige weitere Anordnungen, auch von zusätzlichen Sensorpaaren, möglich.

Typische Beispiele sind in den Fig. 8 und 9 gezeigt, wo die Sensorpaare bzw. die dazwischen aufgespannten Stecken praktisch vollständig den für Wehenmessung relevanten Bereich der Bauchdecke überdecken und so in besonders geeigneter Weise nicht nur Bewegungstätigkeit in verschiedenen Bereichen der Bauchdecke erfassen können, sondern auch die Grundlage für eine zeitabhängige Auswertung dieser ortsbezogenen Daten bieten, mit anderen Worten die Voraussetzung schaffen, dass etwa Wehen, die ausschließlich in einem Teilbereich der Bauchdecke stattfinden und sich mithin auch nur in einer deutlich begrenzenden Bewegung äußern, von Bewegungsmustern unterschieden werden können, die in einem Teilbereich beginnen und sich dann über die gesamte Bauchdecke erstrecken, also als aufeinanderfolgende Signale benachbarter Sensoreinheiten im Zeitverlauf zu beobachten sind.

Auf diese Weise ermöglicht es die vorliegende Erfindung, in bislang ungeahnter Weise nicht nur Wehen als solche zu erkennen, sondern potentiell pathologische bzw. kritische Wehenzustände zu erfassen und diese zuverlässig von Bauchbewegungen zu unterscheiden, die regulärem Wehenverlauf entsprechen. Entsprechend ist eine wie in Fig. 8, Fig. 9 gezeigten Konfigurationen nachgeschaltete Auswerteeinheit mit einer entsprechenden Anzahl von Auswertekanälen versehen, und die üblicherweise mittels digitaler Technik (programmierte Controllereinheiten) realisierte Auswerteelektronik ist in der Lage, diese zeitlichen und/oder räumlichen Muster in geeigneter Weise zu unterscheiden bzw. verschiedenen bestimmten Bewegungsmustern zuzuordnen.

Gemäß einer weiteren, in den Figuren nicht gezeigten Weiterbildung ist der Auswerteeinheit eine bevorzugt automatisierte Darreichungs- bzw. Dosiereinheit nachgeschaltet, die als Reaktion auf eine Auswertung des Wehensignals, insbesondere eine kritische Verringerung der Abstände zwischen einzelnen Wehen, automatisiert die Abgabe eines entsprechenden Medikaments oder Wirkstoffes in die Schwangere einleitet. Dies kann beispielsweise durch ansonsten bekannte Dosierpumpen geschehen, welche im vorliegenden Anwendungsfall etwa mit wehenhemmenden Mitteln oder einem Schmerzmittel befüllt sind und eine vorbestimmte Dosis des Medikaments etwa intravenös abgeben, sobald ein voreinstellbarer, kritischer Zeitabstand zwischen aufeinanderfolgenden Wehen (konkret: zwischen als Wehen erkannten charakteristischen Signaländerungen des Bewegungssignals) unterschritten wird.

Im Ergebnis lässt die vorliegende Erfindung nicht nur Wehentätigkeit zuverlässig überwachen und quantifizieren, auch lassen sich Wehen von weiteren Größen (Atemtätigkeit, Kindsbewegung, Artefakte) trennen, weiterbildungsgemäß können diese weiteren Größen zur zusätzlichen Verwertung herangezogen werden, und in der praktischen Realisierung als portable Ausgabeeinheit (Bezugszeichen 10 in Fig. 3) wird durch die vorliegende Erfindung der Schwangeren ein Höchstmaß an Komfort bei gleichzeitig maximierter Überwachungssicherheit zuteil.

## Patentansprüche

1. Vorrichtung zur Erzeugung von Messdaten betreffend Bewegungen der Bauchdecke (11) einer Person, mit mindestens einem ersten Paar von Sensoreinheiten (12, 14), die zum lösbaren Befestigen auf der Haut jeweils an einem Bauchbereich (13, 15) der Person, beabstandet um einen ersten Abstand (L1), ausgebildet sind,
und einer mit dem ersten Paar von Sensoreinheiten verbundenen Messeinrichtung (20), die zum Erfassen elektrisch auswertbarer Signale der Sensoreinheiten sowie zum Erzeugen eines dem ersten Abstand (L1) sowie Änderungen desselben entsprechenden ersten Abstandssignals ausgebildet ist, wobei
der Messeinrichtung eine Auswerteeinheit (22, 28) nachgeschaltet ist, die zum Auswerten impuls- und/oder wellenförmiger Änderungen des ersten Abstandssignals dergestalt ausgebildet ist, dass nicht-periodische oder periodische Signaländerungen einer Dauer von mindestens 10 Sekunden erfasst, von periodischen, durch eine menschliche Atmung bestimmten Änderungen frequenz-, amplituden- oder signalformmäßig unterschieden und als elektronisch anzeig- oder weiter auswertbares Anzeigesignal ausgegeben werden können, und wobei Signalformerfassungsmittel vorgesehen sind, die, zur Abgrenzung von anderen Signalen, zum Erkennen einer symmetrischen Signalform im Hinblick auf das Signalzeitdiagramm des ersten Abstandssignals ausgebildet sind.

2. Vorrichtung nach Anspruch 1, **gekennzeichnet durch** mindestens ein zweites Paar (16, 18) von Sensoreinheiten, die zum lösbaren Befestigen auf der Haut an dem Bauchbereich der Person, beabstandet um einen zweiten Abstand (L2), ausgebildet sind,
wobei eine dem ersten Abstand (L1) entsprechende, das erste Paar von Sensoreinheiten verbindende erste Strecke in einem vorbestimmten Winkel zu einer dem zweiten Abstand entsprechenden, das zweite Paar von Sensoreinheiten verbindenden zweiten Strecke angeordnet ist.

3. Vorrichtung nach Anspruch 2, **dadurch gekennzeichnet, dass** der vorbestimmte Winkel größer als 0° ist und bevorzugt ein rechter Winkel ist.

4. Vorrichtung nach Anspruch 2 oder 3, **dadurch gekennzeichnet, dass** das erste und das zweite Paar von Sensoreinheiten so befestigbar vorgesehen sind, dass die erste Strecke und die zweite Strecke sich auf dem Bauchbereich (11) kreuzen.

5. Vorrichtung nach einem der Ansprüche 2 bis 4, **dadurch gekennzeichnet, dass** die Auswerteeinheit zur Summen- oder Differenzbildung des ersten Abstandssignals mit einem vom zweiten Paar von Sensoreinheiten ausgegebenen zweiten Abstandssignal ausgebildet ist.

6. Vorrichtung nach einem der Ansprüche 2 bis 5, **gekennzeichnet durch** Mittel zur Erfassung einer räumlichen und zeitlichen Ausbreitung einer Bewegung der Bauchdecke aus einer Auswertung des ersten Abstandssignals sowie mindestens eines von dem mindestens einen zweiten Paar von Sensoreinheiten ausgegebenen zweiten Abstandssignals.

7. Vorrichtung nach einem der Ansprüche 1 bis 6, **dadurch gekennzeichnet, dass** die Auswerteeinheit zum zusätzlichen Erzeugen eines Atemanzeigesignals ausgebildet ist, das als Reaktion auf die erfassten, durch die menschliche Atmung frequenz-, amplituden- und/oder signalformmäßig bestimmten Änderungen des ersten Abstandssignals erzeugt werden kann.

8. Vorrichtung nach einem der Ansprüche 1 bis 7, **gekennzeichnet durch** der Auswerteeinheit zugeordnete Analyse- und Speichermittel (26), die zum elektronischen Speichern des Anzeigesignals sowie zum Erfassen eines Zeitabstandes zwischen aufeinanderfolgenden Anzeigesignalen ausgebildet ist.

9. Vorrichtung nach Anspruch 8, **gekennzeichnet durch** eine den Analyse- und Speichermitteln zugeordnete Alarmeinheit, die zum Vergleichen des erfassten Zeitabstandes mit einem vorbestimmten, voreingestellten Zeitwert sowie zum Ausgeben eines Alarmsignals für den Fall ausgebildet ist, dass eine vorbestimmte Anzahl von aufeinanderfolgenden erfassten Zeitabständen den voreingestellten Zeitwert unterschreitet.

10. Vorrichtung nach einem der Ansprüche 1 bis 9, **dadurch gekennzeichnet, dass** die Messeinrichtung und die Auswerteeinheit Bestandteile einer portablen, insbesondere batteriebetriebenen Einheit (10) sind, die bevorzugt mittels einer drahtlosen Datenverbindung (24) zur Übertragung des Anzeigesignals und/oder weiterer Signale mit einer stationären Basiseinheit verbindbar ist.

11. Vorrichtung nach einem der Ansprüche 1 bis 10, **dadurch gekennzeichnet, dass** die Auswerteeinheit zusätzlich zum Erfassen einer charakteristischen Änderung des ersten und/oder zweiten Abstandsignals ausgebildet ist, die durch eine fetale Kindsbewegung im Bauch der Person hervorgerufen wird und sich durch eine charakteristische Bewegung der Bauchdecke äußert.

12. Vorrichtung nach einem der Ansprüche 1 bis 11, **gekennzeichnet durch** Mittel zur automatisierten Darreichung eines Medikaments, insbesondere zum intravenösen Einleiten des Medikaments in ein Gefäß der Person, wobei die Einleitungsmittel mit der Auswerteeinheit verbunden und so ausgebildet sind, dass als Reaktion auf das Unterschreiten eines vorbestimmten, voreingestellten Zeitabstands aufeinanderfolgender Signaländerungen eine Medikamentendarreichung, insbesondere von wehenhemmenden und/oder Schmerzmitteln, ausgelöst werden kann.

## Claims

1. A device for generating measurement data relating to movements of the abdominal wall (11) of a person, comprising at least one first pair of sensor units (12, 14) which are each configured for detachable fixing to the skin on an abdominal area (13, 15) of the person, spaced apart by a first distance (L1), and a measuring device (20) connected to the first pair of sensor units and configured for detecting signals of the sensor units which can be electrically evaluated, and for generating a first distance signal corresponding to the first distance (L1) and changes thereto,
wherein an evaluation unit (22, 28) is connected to the output of said measuring device and is configured for evaluating pulsed and/or wave-form changes in the first distance signal, in such a way that non-periodic or periodic signal changes lasting at least 10 seconds can be detected, can be distinguished from periodic changes caused by human breathing according to their frequency, amplitude and/or signal form, and can be output in the form of a display signal which can be electronically displayed or evaluated further, and wherein signal-form-detecting means are provided which, to differentiate from other signals, is configured to detect a signal form symmetrical with respect to the signal/time diagram of the first distance signal.

2. A device according to Claim 1, **characterized by** at least one second pair (16, 18) of sensor units configured for detachable fixing to the skin on the abdominal area of the person and spaced apart by a second distance (L2), a first line corresponding to the first distance (L1) and connecting the first pair of sensor units being arranged at a predetermined angle to a second line corresponding to the second distance and connecting the second pair of sensor units.

3. A device according to Claim 2, **characterized in that** the predetermined angle is greater than 0° and is preferably a right-angle.

4. A device according to Claim 2 or 3, **characterized in that** the first and second pairs of sensor units are provided to be so attachable that the first line and the second line cross on the abdominal area (11).

5. A device according to one of claims 2 to 4, **characterized in that** the evaluation unit is configured for summation or subtraction of the first distance signal with respect to a second distance signal output by the second pair of sensor units.

6. A device according to one of claims 2 to 5, **characterized by** means for detecting a spatial and temporal expansion of a movement of the abdominal wall from an evaluation of the first distance signal and at least one second distance signal output by the at least one second pair of sensor units.

7. A device according to one of claims 1 to 6, **characterized in that** the evaluation unit is configured for additional generation of a breathing display signal which can be generated as a reaction to changes of the first distance signal determined by human breathing detected according to frequency, amplitude and/or signal form.

8. A device according to one of claims 1 to 7, **characterized by** analysis and storage means (26) associated with the evaluation unit and configured for electronic storage of the display signal and for detecting a time interval between successive display signals.

9. A device according to Claim 8, **characterized by** an alarm unit associated with the analysis and storage means which is configured for comparing the detected time interval with a predetermined, preset time value and for outputting an alarm signal if a predefined number of successive detected time intervals falls below the preset time value.

10. A device according to one of claims 1 to 9, **characterized in that** the measuring device and the evaluation unit are components of a portable, in particular battery-operated unit (10) which is preferably connectable, by means of a wireless data connection (24), for transmission of the display signal and/or further signals to a stationary base unit.

11. A device according to one of claims 1 to 10, **characterized in that** the evaluation unit is additionally configured for detection of a characteristic change in the first and/or second distance signal caused by a foetal child movement in the abdomen of the person and manifested by a characteristic movement of the abdominal wall.

12. A device according to one of claims 1 to 11, **characterized by** means for the automated dispensing of a medication, in particular for intravenous introduction of the medication into a vessel of the person, the introduction means being connected to the evaluation unit and being so configured that administering of a medication, in particular contraction-inhibiting medication and/or analgesic, can be triggered as a reaction to the case when successive signal changes fall below a predetermined, preset time interval.

## Revendications

1. Dispositif pour créer des données de mesure concernant les mouvements de la paroi abdominale (11) d'une personne, avec au moins une première paire d'unités de capteur (12, 14), qui sont réalisées pour une fixation amovible sur la peau chaque fois sur une zone abdominale (13, 15) de la personne, espacées d'une première distance d'espacement (L1),
et un dispositif de mesure (20) relié à la première paire d'unités de capteur, réalisé pour détecter des signaux évaluables électriquement, provenant des unités de capteur ainsi que pour créer un premier signal d'espacement correspondant au premier espacement (L1) ainsi qu'à ses fluctuations, où
en aval du dispositif de mesure est branchée une unité d'évaluation (22, 28) réalisée pour évaluer des modifications en forme d'impulsions et/ou d'ondes du premier signal d'espacement de manière que des fluctuations de signal non périodiques ou périodiques d'une durée d'au moins 10 secondes soient détectées, soient distinguées en termes de fréquence, d'amplitude ou de forme de signal, vis-à-vis de fluctuations périodiques déterminées par une respiration humaine et puissent être éditées sous forme de signal d'affichage pouvant être affiché ou faire l'objet d'une évaluation supplémentaire, par voie électronique, et où
sont prévus des moyens de détection de forme de signal qui, pour obtenir une délimitation vis-à-vis d'autres signaux, sont réalisés pour identifier une forme de signal symétrique eu égard au chronogramme du premier signal d'espacement.

2. Dispositif selon la revendication 1, **caractérisé par** au moins une deuxième paire (16, 18) d'unités de capteur, qui sont réalisées pour une fixation amovible sur la peau de la zone abdominale de la personne, espacées d'une deuxième distance d'espacement (L2),
un premier chemin, correspondant à la première distance d'espacement (L1), reliant la première paire d'unités de capteur, étant disposé sous un angle prédéterminé par rapport à un deuxième chemin, correspondant à la deuxième distance d'espacement, reliant la deuxième paire d'imités de capteur.

3. Dispositif selon la revendication 2, **caractérisé en ce que** l'angle prédéterminé est supérieur à 0° et est, de préférence, un angle droit.

4. Dispositif selon la revendication 2 ou 3, **caractérisé en ce que** la première et la deuxième paire d'unités de capteur sont prévues de façon à pouvoir être fixées de manière que le premier chemin et le deuxième chemin se croisent sur la zone abdominale (11).

5. Dispositif selon l'une des revendications 2 à 4, **caractérisé en ce que** l'unité d'évaluation est réalisée pour former la somme ou la différence du premier signal d'espacement avec un deuxième signal d'espacement produit par la deuxième paire d'unités de capteur.

6. Dispositif selon l'une des revendications 2 à 5, **caractérisé par** des moyens pour détecter une propagation spatiale et temporelle d'un déplacement de la paroi abdominale à partir d'une évaluation du premier signal d'espacement ainsi qu'au moins un deuxième signal d'espacement émis par la au moins une deuxième paire d'unités de capteur.

7. Dispositif selon l'une des revendications 1 à 6, **caractérisé en ce que** l'unité d'évaluation est réalisée pour créer en plus un signal d'affichage respiratoire qui peut être créé en réaction aux fluctuations du premier signal d'espacement, fluctuations détectées, déterminées en termes de fréquence d'amplitude et/ou de forme de signal, par la respiration humaine.

8. Dispositif selon l'une des revendications 1 à 7, **caractérisé par** des moyens d'analyse et de stockage (26) associés à l'unité d'évaluation, réalisés pour le stockage électronique du signal d'affichage ainsi que pour la détection d'un espacement temporel entre des signaux d'affichage successifs.

9. Dispositif selon la revendication 8, **caractérisé par** une unité d'alarme associée aux moyens d'analyse et de stockage, unité d'alarme réalisée pour comparer l'espacement temporel détecté à une valeur temporelle préréglée, prédéterminée ainsi que pour émettre un signal d'alarme dans le cas où un nombre prédéterminé d'espacements temporels détectés successifs descend au-dessous de la valeur temporelle préréglée.

10. Dispositif selon l'une des revendications 1 à 9, **caractérisé en ce que** le dispositif de mesure et l'unité d'évaluation sont des composants d'une unité (10) portable en particulier fonctionnant sur batterie, pouvant être combinée à une unité de base stationnaire, de préférence, à l'aide d'une liaison de données (24) sans fil afin de transmettre le signal d'affichage et/ou d'autres signaux.

11. Dispositif selon l'une des revendications 1 à 10, **caractérisé en ce que** l'unité d'évaluation est en plus réalisée pour détecter une fluctuation caractéristique du premier et/ou du deuxième signal d'espacement, provoquée par un mouvement foetal d'un enfant dans le ventre de la personne et s'exprimant par un mouvement caractéristique de la paroi abdominale.

12. Dispositif selon l'une des revendications 1 à 11, **caractérisé par** des moyens pour administrer de façon automatisée un médicament, en particulier pour l'injection intraveineuse du médicament dans un vaisseau de la personne, dans lequel les moyens d'injection sont reliés à l'unité d'évaluation et sont réalisés de manière qu'en réaction au fait que des variations de signaux successifs descendent au-dessous d'un espacement temporel préréglé, prédéterminé, peut être déclenché l'administration d'un médicament, en particulier d'agents contre les douleurs de l'accouchement et/ou contre les autres douleurs.
